# EUROPÄISCHE PATENTSCHRIFT

(11) **EP 1 262 147 B1**
(45) Veröffentlichungstag und Bekanntmachung des Hinweises auf die Patenterteilung: **10.11.2004**
(21) Anmeldenummer: 02100571.5
(22) Anmeldetag: 28.05.2002
(51) Int. Cl.: A61B 6/06, H05G 1/64

(54) **Vorrichtung und Verfahren zur Anpassung der Strahlungsdosis einer Röntgenstrahlungsquelle**
Device and method for adjusting the radiation dose from an X-ray source
Appareil et méthode d'ajustement d'une dose de rayonnement d'une source de rayons X

(30) Priorität: 31.05.2001 DE 10132816
(43) Veröffentlichungstag der Anmeldung: 04.12.2002
(73) Patentinhaber: Philips Intellectual Property & Standards GmbH, 20099 Hamburg (DE); Koninklijke Philips Electronics N.V., 5621 BA Eindhoven (NL)
(72) Erfinder: Braes, Henning c/o Philips Corp. Intel Prop. GmbH,, 52066, Aachen (DE); Schmitz, Georg, Dr., 52066, Aachen (DE); Reiter, Harald c/o Philips Corp. Intel. Prop. GmbH, 52066, Aachen (DE)
(74) Vertreter: Volmer, Georg, Dipl.-Ing.

(56) Entgegenhaltungen:
- US-A- 5 664 000
- US-A- 6 084 940
- US-A- 6 148 060
- US-B1- 6 175 614

## Beschreibung

Die Erfindung betrifft ein Verfahren zur Anpassung der Strahlungsdosis einer Röntgenstrahlungsquelle, welche ein zu untersuchendes Objekt durchstrahlt und dabei ein Durchleuchtungsbild des Objektes erzeugt. Ferner betrifft die Erfindung eine Röntgenvorrichtung zur Durchführung des genannten Verfahrens.

Zur Aufnahme von Röntgen-Durchleuchtungsbildern wird ein zu untersuchendes Obj ekt wie etwa bei industriellen Anwendungen ein Werkstück oder bei medizinischen Anwendungen der Körper eines Patienten mit Röntgenstrahlung durchleuchtet. Die transmittierte Röntgenstrahlung wird dann von einem bildgebenden Detektor aufgezeichnet und in ein Abbild der Absorptionsstärkeverteilung umgewandelt. Dabei tritt häufig die Situation auf, dass nur ein Teil des Durchleuchtungsbildes für den zugrunde liegenden Untersuchungszweck interessiert. Die Strahlungsdosis der Röntgenstrahlungsquelle sollte demnach so eingestellt werden, dass dieser interessierende Bereich optimal abgebildet wird. Bei medizinischen Anwendungen liegen nicht-interessierende Bildbereiche insbesondere dann vor, wenn Röntgenstrahlung am Körper des Patienten vorbei direkt auf den Detektor fällt ("Direktstrahlung") und daher keine Information über die Absorption des Gewebes trägt. Ebenso sind Bildbereiche uninteressant, welche aufgrund einer Ausblendung durch Absorptionsfilter keine Röntgenstrahlung empfangen.

Zur Anpassung der Röntgenstrahlungsdosis an das Durchleuchtungsbild ist es bekannt, die Gesamtdosis zu messen, die im gesamten Messfeld des Röntgendetektors ankommt. Wenn dabei in einem gewissen, typischerweise kleinen Teil des Messfeldes Direktstrahlung auftritt, wird eine hohe Gesamtdosis gemessen, welche dazu führt, dass die Röntgenstrahlungsdosis niedriger geregelt wird als nötig. Zur Vermeidung dieses Effektes ist es aus der WO 98 /48600 bekannt, das Histogramm der Grauwerteverteilung des aufgenommenen Durchleuchtungsbildes zu bestimmen und hieraus nach vorgegebenen Kriterien, die zum Beispiel durch Fuzzy-Logic-Regeln definiert sein können, einen Grawert-Schwellwerd festzulegen.

Alle Bildpunkte mit einem Grauwert unterhalb des festgelegten Schwellwertes sollen dann definitionsgemäß zum interessierenden Bildbereich gehören. Die Anpassung der Röntgenstrahlungsdosis erfolgt anschließend nur unter Berücksichtigung des interessierenden Bildbereiches, das heißt der Punkte, deren Grauwert unter dem genannten Schwellwert liegt. Nachteilig bei diesem Verfahren ist, dass allein der Grauwert eines Bildpunktes darüber entscheidet, ob dieser Bildpunkt zum interessierenden Bildbereich gehört oder nicht. Dies kann jedoch insbesondere bei isoliert liegenden Punkten mit abweichenden Grauwerten dazu führen, dass diese falsch in Bezug auf den interessierenden Bildbereich zugeordnet werden.

Aus der US-Patentschrift US 6,148,060 ist ein Verfahren zur automatischen Steuerung der Dosis einer Röntgenröhre bekannt, bei dem ein Durchleuchtungsbild zuerst in Zellen unterteilt wird, worauf aus den Zellen durch bedingtes Zusammenfassen Bildregionen gebildet werden, und aus den Bildregionen interessierende Bildregionen ausgewählt werden, deren Durchschnittsgrauwerte ein vorgegebenes Kriterium erfüllen müssen.

Vor diesem Hintergrund war es eine Aufgabe der vorliegenden Erfindung, ein Verfahren und eine Vorrichtung zur Anpassung einer Strahlungsdosis einer Röntgenstrahlungsquelle bereitzustellen, welche eine korrektere Anpassung an die interessierenden Bildbereiche erlauben.

Diese Aufgabe wird durch ein Verfahren mit den Merkmalen des Anspruchs 1 sowie eine Röntgenvorrichtung mit den Merkmalen des Anspruchs 4 gelöst. Vorteilhafte Ausgestaltungen sind in den Unteransprüchen enthalten.

Das Verfahren dient der Anpassung der Strahlungsdosis einer Röntgenstrahlungsquelle, welche ein zu untersuchendes Objekt wie zum Beispiel den Körper eines Patienten durchstrahlt und dabei ein Durchleuchtungsbild des Objektes erzeugt. Bei dem Verfahren wird das Durchleuchtungsbild in zusammenhängende Bildregionen einer vorgegebenen Mindestgröße unterteilt, und die Strahlungsdosis wird so angepasst, dass sie den interessierenden Bildregionen optimal angepasst ist. Die interessierenden Bildregionen werden dabei dadurch definiert, dass ihr Durchschnittsgrauwert ein vorgegebenes Kriterium erfüllt.

Anders als bei den bekannten Verfahren wird bei dem geschilderten Vorgehen der interessierende Bildbereich nicht Bildpunkt für Bildpunkt festgelegt, sondern aus Bildregionen mit einer vorgegebenen Mindestgröße, das heißt einer minimalen Anzahl von Bildpunkten, zusammengesetzt. Dieses Vorgehen garantiert, dass der interessierende Bildbereich aus zusammenhängenden Stücken besteht, und dass nicht etwa isolierte Inseln von nur einem oder nur wenigen Bildpunkten zum Bildbereich gehören beziehungsweise dass umgekehrt nicht kleine Löcher von nur einem oder wenigen Bildpunkten im Bildbereich existieren. Das Verfahren erzielt hierdurch eine realistischere Festlegung des interessierenden Bildbereiches, so dass die nachfolgende Anpassung der Röntgenstrahlungsdosis auf der Basis dieses interessierenden Bildbereiches ein besseres Ergebnis liefert. Ferner ist von Vorteil, dass die Auswertung von Bildregionen einer bestimmten Mindestgröße in der Regel schneller erfolgen kann als die Auswertung aller Bildpunkte einzeln. Typischerweise liegt die Mindestgröße der Bildregionen bei ca. 100 bis 100.000 Bildpunkten (Pixeln).

Für die Unterteilung des Durchleuchtungsbildes in zusammenhängende Bildregionen einer vorgegebenen Mindestgröße gibt es verschiedene Möglichkeiten. Im einfachsten Falle wird das Durchleuchtungsbild in ein regelmäßiges Raster aus rechteckigen Bildregionen unterteilt, wobei die Bildregionen alle die gleiche Größe haben können. Da in vielen Fällen die wahrscheinlichste Lage für das Auftreten einer Grenze des interessierenden Bildbereiches bekannt ist, kann die Größe der Bildregionen in einem solchen Grenzbereich auch kleiner vorgegeben werden, um eine bessere Auflösung des Grenzverlaufes zu erreichen.

Als Ausgestaltung des genannten Kriteriums werden die (potentiell) interessierenden Bildregionen ausgehend von der Menge aller Bildregionen iterativ bestimmt, indem gemäß den folgenden Schritten nach und nach nicht-interessierende Bildregionen abgesondert werden:
a) Absonderung der hellsten Bildregion von der Menge der potentiell interessierenden Bildregionen;
b) Bestimmung des Durchschnittsgrauwertes der in Schritt a) abgesonderten Bildregion;
c) Bestimmung des Durchschnittsgrauwertes aller nach der Absonderung in Schritt a) noch verbleibenden potentiell interessierenden Bildregionen;
d) falls das Paar der in den Schritten b) und c) bestimmten Durchschnittsgrauwerte außerhalb einer vorgegebenen charakteristischen Menge von Wertepaaren liegt, wird die Iteration erneut bei Schritt a) begonnen, wobei die zuvor abgesonderte Bildregion nicht mehr zur Menge der potentiell interessierenden Bildregionen gehört;
falls das Paar der in den Schritten b) und c) bestimmten Durchschnittsgrauwerte dagegen innerhalb der genannten charakteristischen Menge liegt, ist die Iteration beendet, und die Menge der interessierenden Bildregionen wird mit der Menge der potentiell interessierenden Bildregionen vor der letzten Ausführung von Schritt a) identifiziert.

Durch das geschilderte iterative Verfahren ist es in einfacher Weise möglich, die interessierenden Bildregionen zu bestimmen und dabei solche Bildregionen auszuschließen, die zum Beispiel aufgrund von Direktstrahlung einen sehr hohen Durchschnittsgrauwert aufweisen. Die Anpassung der Dosis der Röntgenstrahlungsquelle ist somit auch bei Vorhandensein von Direktstrahlung möglich, wobei zur Dosisregelung keine detaillierten Kenntnisse der Systemparameter erforderlich sind.

Gemäß einer bevorzugten Ausgestaltung des Verfahrens werden zu einer Bildregion jeweils alle zusammenhängenden Punkte des örtlich tiefpassgefilterten Durchleuchtungsbildes zusammengefasst, deren Grauwerte in einem vorgegebenen Intervall liegen. Der "Zusammenhang" der Punkte bedeutet, dass die Bildregion eine geometrisch zusammenhängende Fläche bildet, so dass jedes Punktepaar der Bildregion durch eine in der Bildregion verlaufende Linie verbunden werden kann. Die Tiefpassfilterung des Durchleuchtungsbildes stellt sicher, dass große Helligkeitssprünge ausgeglichen beziehungsweise "verschmiert" werden, so dass isolierte Bildpunkte mit abweichender Helligkeit vermieden werden.

Die charakteristische Menge, welche in Schritt d) des oben erläuterten iterativen Verfahrens zur Unterscheidung zwischen nicht-interessierenden und interessierenden Bildregionen verwendet wird, kann auf verschiedene Art und Weise festgelegt und insbesondere experimentell bestimmt werden. Vorzugsweise wird diese charakteristische Menge so definiert, dass das Paar der in den Schritten b) und c) bestimmten Durchschnittsgrauwerte außerhalb dieser charakteristischen Menge liegt, wenn das Verhältnis des Durchschnittsgrauwertes der abgesonderten Bildregion zum Durchschnittsgrauwert der verbleibenden, potentiell interessierenden Bildregionen oberhalb eines Schwellwertes liegt. Dieser Schwellwert kann typischerweise im Bereich von 1,5 : 1 bis 10: 1 liegen.

Die Erfindung betrifft weiterhin eine Röntgenvorrichtung, welche die folgenden Elemente enthält:
- eine Röntgenstrahlungsquelle zur Abgabe von Röntgenstrahlung;
- einen Röntgendetektor zur Erzeugung eines Durchleuchtungsbildes eines von der Röntgenstrahlungsquelle durchstrahlten Objektes;
- eine Regdungseinheit zur Anpassung der Strahlungsdosis der Röntgenstrahlungsquelle, wobei die Regelungseinheit so eingerichtet ist, dass sie ein Verfahren der oben erläuterten Art ausführen kann.

Eine derartige Röntgenvorrichtung gewährleistet eine realistische Festlegung eines interessierenden Bildbereiches, welcher zur Anpassung der Strahlungsdosis der Röntgenstrahlungsquelle herangezogen wird Damit ist die Röntgenvorrichtung in der Lage, qualitativ bessere Durchleuchtungsbilder von zu untersuchenden Objekten zu liefern.

Im Folgenden wird die Erfindung mit Hilfe der Figuren beispielhaft erläutert. Es zeigt:
Fig 1 schematisch die Komponenten einer erfindungsgemäßen Röntgenvorrichtung sowie ein in Bildregionen unterteiltes Durchleuchtungsbild;
Fig 2 ein Flussdiagramm des iterativen Verfahrens zur Festlegung der interessierenden Bildregionen.

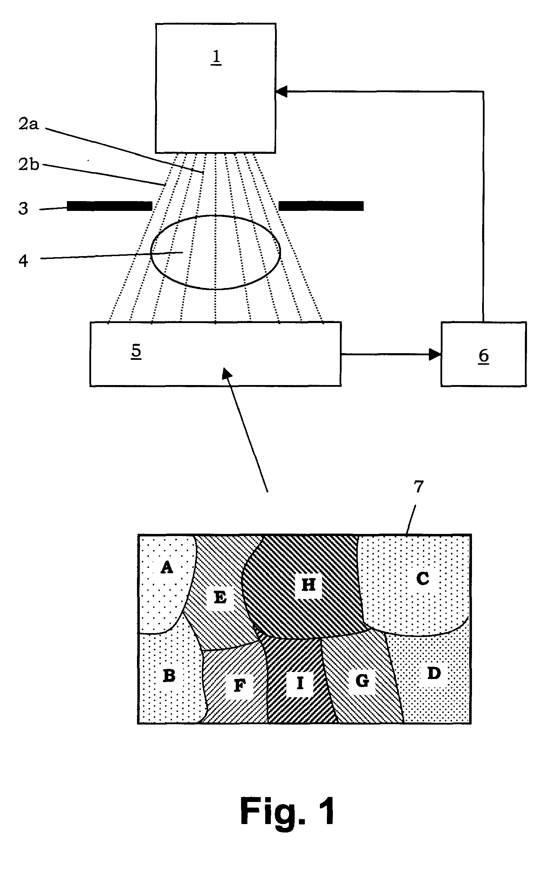

In Figur 1 sind schematisch die Komponenten einer Röntgenvorrichtung dargestellt, welche in einer medizinischen Anwendung zur Erzeugung eines Durchleuchtungsbildes eines menschlichen Körpers 4 dient. Die Röntgenvorrichtung besteht aus einer Röntgenstrahlungsquelle 1, welche im Betrieb Röntgenstrahlung in Richtung des Patienten 4 aussendet. Dabei trifft ein erster Teil 2a dieser Röntgenstrahlung auf den Körper 4 und durchstrahlt ihn, während im seitlichen Bereich ein Teil 2b der Strahlung am Körper 4 vorbei verläuft. Diese Strahlung 2b trifft in dem unter dem Patienten 4 angeordneten Röntgendetektor 5 als sogenannte Direktstrahlung auf und enthält keine Information über das zu untersuchende Objekt. Durch Absorberfilter 3 kann versucht werden, die Größe der Direktstrahlung 2b zu reduzieren, was in der Regel jedoch nicht lückenlos gelingt.

Der Röntgendetektor 5 ist unter anderem mit einer Regelungseinheit 6 verbunden, welche wiederum ausgangsseitig mit der Röntgenstrahlungsquelle 1 gekoppelt ist. Die Regdungseinheit 6 wertet das vom Röntgendetektor 5 produzierte Durchleuchtungsbild aus und passt die von der Röntgenstrahlungsquelle 1 abgegebene Strahlungsdosis so an, dass sie einen für die Darstellung des interessierenden Bildbereiches des Durchleuchtungsbildes optimalen Wert aufseist. Dabei ist in der Regelungseinheit 6, bei der es sich typischerweise um eine elektronische Datenverarbeitungseinheit handelt, das nachfolgend detaillierter erläuterte Verfahren implementiert.

Bei dem genannten Verfahren wird das vom Röntgendetektor 5 produzierte Durchleuchtungsbild 7, welches in Figur 1 schematisch dargestellt ist, zunächst in verschiedene Regionen A-I unterteilt. Im einfachsten Falle können diese durch eine schachbrettartige Unterteilung des Durchleuchtungsbildes 7 in rechteckige Bildregionen gleicher Größe erzeugt werden. Alternativ können die Bildregionen A- I anhand der dort vorliegenden Grauwerte der einzelnen Bildpunkte festgelegt werden, wobei in einer Bildregion alle miteinander in Kontakt stehenden Bildpunkte zusammengefasst werden, deren Grauwert in einem dieser Bildregion zugeordneten Intervall liegt. Die Bildregionen weisen ferner jeweils eine gewisse Mindestgröße auf, das heißt eine Mindestanzahl von Bildpunkten. Ein weiteres mögliches Verfahren zur Unterteilung des Durchleuchtungsbildes ist in R. Mester, T. Aach, U. Franke: Image Segmentation Using Likelihood Ratio Tests and Markov Region Shape Models, Signal Processing IV: Theories and Applications, EUSIPCO 88, Grenoble, France (Herausgeber: J. L. Lacoume, A. Chehikian, N. Martin, J. Malbos, Seiten 104-110, September 1988) beschrieben.

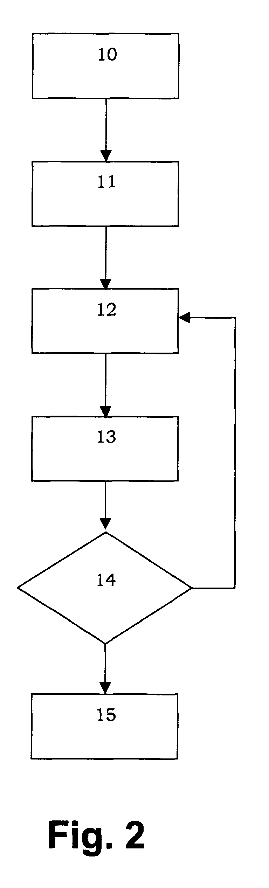

Sodann werden in einem iterativen Verfahren nach und nach Bildregionen abgesondert, deren Helligkeit so groß ist, dass sie auf das Vorliegen von Direktstrahlung in dieser Bildregion hinweist. Dieses iterative Verfahren wird nachfolgend mit Hilfe des Flussdiagramms von Figur 2 erläutert.

Im ersten Schritt 10 wird wie bereits erläutert das gesamte Durchleuchtungsbild 7 in die Bildregionen A- I unterteilt. Im nächsten Schritt 11 werden die Bildregionen dann entsprechend ihrem Durchschnittsgrauwert sortiert, wobei angenommen sei, dass in Figur 1 die Zuordnung der Buchstaben A-I zu den Bildregionen in der alphabetischen Reihenfolge entsprechend den Durchschnittsgrauwerten dieser Bildregionen (beginnend beim hellsten Grauwert) erfolgt sei.

Die eigentliche Iterationsschleife besteht aus den Schritten 12, 13 und 14. Während der Iterationsschleife wird eine Menge potentiell interessierender Bildregionen sukzessive verkleinert. Zu Beginn der Schleife besteht die Menge der potentiell interessierenden Bildregionen aus allen Bildregionen A - I des Durchleuchtungsbildes 7.

Bei der ersten Ausführung des ersten Schrittes 12 der Iteration wird diejenige Bildregion A aus der Menge der potentiell interessierenden Bildregionen abgesondert, die den größten Durchschnittsgrauwert unter allen Bildregionen A - I aufweist.

Im nächsten Schritt 13 werden der Durchschnittsgrauwert DGW1 der abgesonderten Bildregion A sowie der Durchschnittsgrauwert DGW2 der verbleibenden Bildregionen B-I (d.h. der aktuellen Menge der potentiell interessierenden Bildregionen) berechnet.

Im Schritt 14 werden die berechneten Durchschnittsgrauwerte DGW1 und DGW2 dazu verwendet zu entscheiden, ob die abgesonderte Bildregion A Direktstrahlung enthält oder nicht. Das Kriterium für die Annahme von Direktstrahlung kann etwa darin bestehen, dass das Verhältnis der Durchschnittsgrauwerte DGW1 : DGW2 oberhalb eines vorgegebenen Schwellwertes von zum Beispiel 2 : 1 liegt.

Falls Schritt 14 ergibt, dass die abgesonderte Bildregion A Direktstrahlung enthält, wird sie definitiv von der Menge der potentiell interessierenden Bildregionen abgesondert, und das Verfahren wird bei Schritt 12 mit den verbleibenden Bildregionen B - I fortgesetzt. Bei der zweiten Ausführung des Schrittes 12 wird dann die nächste hellste Bildregion B abgesondert und so weiter.

Wenn dagegen in Schritt 14 festgestellt wird, dass die bei der letzten Ausführung des Schrittes 12 abgesonderte Bildregion kein Direktlicht enthält, wird diese Bildregion wieder den verbleibenden, potentiell interessierenden Bildregionen hinzugefügt, und im Schritt 15 wird der interessierende Bildbereich aus diesen potentiell interessierenden Bildregionen gebildet, welche kein Direktlicht enthalten.

Für das in Figur 1 schematisch dargestellte Durchleuchtungsbild 7 würden zum Beispiel bei viermaligem Durchlaufen der Schleife 12 - 14 die Bildregionen A, B, C und D als Regionen mit Direktstrahlung abgesondert und der interessierende Bildbereich aus den Bildregionen E, F, G, H und I zusammengesetzt.

## Patentansprüche

1. Verfahren zur Anpassung der Strahlungsdosis einer Röntgenstrahlungsquelle, welche ein zu untersuchendes Objekt durchstrahlt und dabei ein Durchleuchtungsbild des Objektes erzeugt, wobei das Durchleuchtungsbild in zusammenhängende Bildregionen einer vorgegebenen Mindestgröße unterteilt und die Strahlungsdosis so angepasst wird, dass sie den interessierenden Bildregionen, deren Durchschnittsgrauwert ein vorgegebenes Kriterium erfüllt, optimal angepasst ist, **dadurch gekennzeichnet,**
**dass** das vorgegebene Kriterium für die interessierenden Bildregionen ausgehend von der Menge aller Bildregionen iterativ gemäß den folgenden Schritten angewendet wird:
a) Absonderung der hellsten Bildregion;
b) Bestimmung des Durchschnittsgrauwertes der abgesonderten Bildregion;
c) Bestimmung des Durchschnittsgrauwertes der verbleibenden Bildregionen;
d) erneuter Beginn bei Schritt a) ausgehend von der Menge der verbleibenden Bildregionen, wenn das Paar der in den Schritten b) und c) bestimmten Durchschnittsgrauwerte außerhalb einer vorgegebenen charakteristischen Menge liegt; andernfalls Identifikation der verbleibenden Bildregionen und der zuletzt abgesonderten Bildregion mit der Menge der interessierenden Bildregionen.

2. Verfahren nach Anspruch 1,
**dadurch gekennzeichnet,**
**dass** jeweils alle zusammenhängenden Punkte des tiefpassgefilterten Durchleuchtungsbildes zu einer Bildregion zusammengefasst werden, deren Grauwerte in einem vorgegebenen, zugeordneten Intervall liegen.

3. Verfahren nach Anspruch 1,
**dadurch gekennzeichnet,**
**dass** das Paar der in den Schritten b) und c) bestimmten Durchschnittsgrauwerte dann außerhalb einer vorgegebenen charakteristischen Menge liegt, wenn das Verhältnis des Durchschnittsgrauwertes der abgesonderten Bildregion zum Durchschnittsgrauwert der verbleibenden Bildregionen oberhalb eines Schwellwertes liegt.

4. Röntgenvorrichtung, enthaltend
- eine Röntgenstrahlungsquelle;
- einen Röntgendetektor zur Erzeugung eines Durchleuchtungsbildes eines von der Röntgenstrahlungsquelle durchstrahlten Objektes;
- eine Regelungseinheit zur Anpassung der Strahlungsdosis der Röntgenstrahlungsquelle, wobei die Regelungseinheit so eingerichtet ist, dass sie ein Verfahren nach mindestens einem der Ansprüche 1 bis 3 ausführen kann.

## Claims

1. A method for adapting the radiation dose of an X-ray source which irradiates an object to be examined so as to form an X-ray image of the object, in which method the X-ray image is subdivided into coherent image regions which have a predetermined minimum format and the radiation dose is adapted so as to be optimum for the image regions of interest whose mean grey value satisfies a predetermined criterion, **characterized in that** the predetermined criterion for the image regions of interest is iteratively applied to the total number of image regions in conformity with the following steps:
a) separating the brightest image region,
b) determining the mean grey value of the separated image region,
c) determining the mean grey value of the remaining image regions,
d) starting again with the step a) on the basis of the number of remaining image regions if the pair of mean grey values determined in the steps b) and c) lies outside a predetermined characteristic number, and otherwise identifying the remaining image regions and the last separated image region as the number of image regions of interest.

2. A method as claimed in claim 1, **characterized in that** all coherent points of the low-pass filtered X-ray whose grey values are within a predetermined, associated interval are combined each time so as to form an image region.

3. A method as claimed in claim 1, **characterized in that** the pair of mean grey values determined in the steps b) and c) lies outside a predetermined characteristic number when the ratio of the mean grey value of the separated image region to the mean grey value of the remaining image regions exceeds a threshold value.

4. An X-ray device which includes
- an X-ray source,
- an X-ray detector for forming an X-ray image of an object irradiated by the X-ray source,
- a control unit for adapting the radiation dose of the X-ray source, the control unit being arranged in such a manner that it is capable of carrying out a method as claimed in at least one of the claims 1 to 3.

## Revendications

1. Procédé d'ajustement de la dose de rayonnement d'une source de rayons X qui traverse un objet à étudier et produit en l'occurrence une image radioscopique de l'objet, l'image radioscopique étant subdivisée en régions d'image cohérentes de dimensions minimales données et la dose de rayonnement étant ajustée de telle sorte que celle-ci soit adaptée de manière optimale aux régions d'image intéressantes dont la valeur de gris moyenne répond à un critère préalablement déterminé, **caractérisé en ce**
**que** le critère préalablement déterminé est utilisé de manière itérative pour les régions d'image intéressantes à partir de la quantité de toutes les régions d'image selon les étapes suivantes:
a) isolement de la région d'image la plus claire;
b) détermination de la valeur de gris moyenne de la région d'image isolée;
c) détermination de la valeur de gris moyenne des régions d'image résiduelles;
d) retour à l'étape a) en partant de la quantité des régions d'image résiduelles lorsque la paire des valeurs de gris moyennes déterminées dans les étapes b) et c) se trouve en dehors d'une quantité caractéristique préalablement déterminée;
sinon, identification des régions d'image résiduelles et de la région d'image isolée en dernier lieu avec la quantité des régions d'image intéressantes.

2. Procédé selon la revendication 1,
**caractérisé en ce**
**que** tous les points cohérents de l'image radioscopique après filtrage passe-bas sont respectivement rassemblés en une région d'image dont les valeurs de gris se trouvent dans un intervalle affecté préalablement déterminé.

3. Procédé selon la revendication 1,
**caractérisé en ce**
**que** la paire des valeurs de gris moyennes déterminées dans les étapes b) et c) se trouve en dehors d'une quantité caractéristique préalablement déterminée lorsque le rapport entre la valeur de gris moyenne de la région d'image isolée et la valeur de gris moyenne des régions d'image résiduelles se trouve au-delà d'une valeur-seuil.

4. Dispositif de radiographie contenant
- une source de rayons X;
- un détecteur de rayons X pour la production d'une image radioscopique d'un objet traversé par la source de rayons X;
- une unité de réglage pour l'adaptation de la dose de rayonnement de la source de rayons X, l'unité de réglage étant organisée de telle sorte qu'elle puisse exécuter un procédé selon au moins l'une des revendications 1 à 3.
